Europäisches Patentamt

European Patent Office

Office européen des brevets

(11) Veröffentlichungsnummer: **0 001 983**
**B1**

(12) **EUROPÄISCHE PATENTSCHRIFT**

(21) Anmeldenummer: 78101182.0

(22) Anmeldetag: 20.10.78

(51) Int. Cl.³: **C 07 C 121/43,**
**C 07 C 120/00**

(54) Verfahren zur Isolierung bzw. Gewinnung von N,N-Dimethylaminoacetonitril aus N,N-Dimethylaminoacetonitril/Wasser-Gemischen

(30) Priorität: 02.11.77 DE 2748964

(43) Veröffentlichungstag der Anmeldung:
30.05.79 Patentblatt 79/11

(45) Bekanntmachung des Hinweises auf die Patenterteilung:
10.12.80 Patentblatt 80/25

(84) Benannte Vertragsstaaten:
CH DE FR GB

(73) Patentinhaber: **BAYER AG**
**Zentralbereich Patente, Marken und Lizenzen**
**D - 5090 Leverkusen 1, Bayerwerk (DE)**

(72) Erfinder: **Lenthe, Manfred, Dr.**
**Michaelshöhe 40**
**D - 5068 Odenthal (DE)**
**Dankert, Gerhard, Dr.**
**A.-Hantzsch-Strasse 44**
**D - 5000 Koeln 80 (DE)**

(56) Entgegenhaltungen:
DE - A - 2 311 572
JOURNAL OF THE AMERICAN CHEMICAL
SOCIETY, vol. 68, August 1946,
Easton, Pa., USA
R. A. TURNER "β-Dimethyl-aminoethylamine und
Dimethylaminoacetonitrile"
Seiten 1607 bis 1608
JUSTUS LIEBIG'S ANNALEN DER CHEMIE,
Band 279,
W. ESCHWEILER "Uber einige Acetonitrile"
1894, Winter'sche Verlag, Leipzig
Seite 39 bis 44

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Isolierung bzw. Gewinnung von N,N-Dimethylaminoacetonitril aus N,N-Dimethylamino-acetonitril/Wasser-Gemischen

Die Erfindung betrifft ein Verfahren zur Gewinnung von N,N-Dimethylaminoacetonitril aus dem bei der Herstellung aus Dimethylamin, Blausäure und Formaldehyd anfallenden Nitril-Wasser-Gemischen durch Extraktion mit 1,2-Dichlorbenzol.

Die Herstellung des N,N-Dimethylaminoacetonitrils aus Dimethylamin, Blausäure und Formaldehyd nach einer lange bekannten Reaktion (Typ der sogenannten "Mannich-Reaktion", vgl. "Organikum", VEB Deutscher Verlag der Wissenschaften, Berlin, 8. Auflage (1968), Seite 447 ff.) gelingt in hohen Ausbeuten. Schwierig ist jedoch die Abtrennung von Wasser von dem Produktgemisch. Reines N,N-Dimethylaminoacetonitril siedet bei Kp 760 mm/137°C und bildet in dem für die technische Destillation geeigneten Druckbereich azeotrope Mischungen mit Wasser, die je etwa 50 Gew.—% der beiden Komponenten enthalten. Aus diesem Grunde wurde zur Isolierung des Nitrils die Extraktion mit Chloroform oder Benzol vorgeschlagen (Verfahren nach R. A. Turner, J. Am. Chem. Soc. *68*, 1607 (1946), von anderen Autoren (Eschweiler, Liebigs Ann. Chem. *279*, 43 (1894)) wurde die Extraktion mit Diäthyläther empfohlen.

Die genannten Extraktionsmittel haben den Nachteil entweder hoher Toxizität (Chloroform, Benzol) oder niedriger Flammpunkte (Benzol, Äther); sie sieden alle niedriger als das zu gewinnende Produkt und müssen daher mit hohem Energieaufwand aus den Extraktionsgemischen destilliert werden.

Es wurde nun gefunden, daß man das N,N-Dimethylaminoacetonitril dann in besonders einfacher Weise erhält, wenn man die aus Dimethylamin, Blausäure und Formaldehyd erhaltene N,N-Dimethylacetonitril/Wasser-Mischung mit 1,2-Dichlorbenzol extrahiert.

Es ist überraschend, daß 1,2-Dichlorbenzol die Nachteile der nach dem Stand der Technik bekannten Extraktionsmittel vermeidet und daher ein für die Praxis überlegenes Lösungsmittel darstellt. Wie sich dabei noch gezeigt hat, läßt sich bei Verwendung von 1,2-Dichlorbenzol die in den meisten Fällen notwendige Abtrennung des mitgeschleppten Wassers aus der Extraktphase leicht durch eine nachfolgende Destillation bei niedrigen Temperaturen bewerkstelligen. Das erfindungsgemäße Verfahren bringt somit einen technischen Fortschritt mit sich.

Die Extraktion des Dimethylaminoacetonitrils gelingt nach dem erfindungsgemäßen Extraktionsverfahren mit hohen Ausbeuten, wenn man bei Temperaturen zwischen 5 und 90°C, vorzugsweise zwischen 15 und 30°C arbeitet und auf 1 Gewichtsteil des N,N-Dimethylamino-acetonitril/Wasser-Gemisches zwischen 1,5 und 10 Gewichtsteilen, vorzugsweise zwischen 2,5 und 5 Gewichtsteilen 1,2-Dichlorbenzol einsetzt.

Das eingesetzte Nitril/Wasser-Gemisch kann salzhaltig, z.B. bedingt durch den Einsatz von Natriumcyanid in der Reaktion zur Bildung des Nitrils, aber auch salzfrei sein, wenn die Reaktion mit Cyanwasserstoff durchgeführt wird.

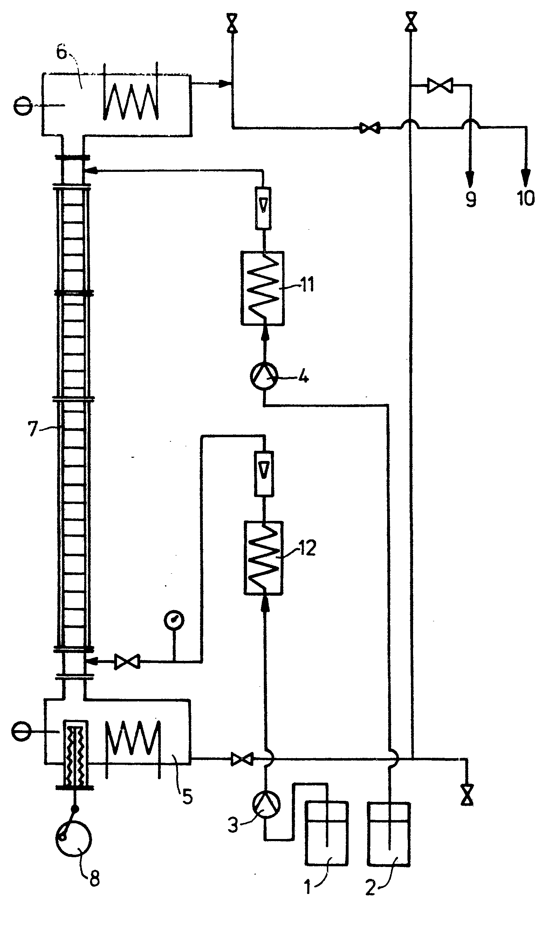

Das Verfahren gliedert sich in eine Extraktion, die in der in der Abbildung aufgezeigten Anlage durchgeführt wird, und in eine nachfolgende Destillation. Bei der Extraktion wird eine wäßrige Phase (praktisch nur aus Wasser bestehend) und eine organische Phase erhalten, letztere besteht hauptsächlich aus dem Lösungsmittel (1,2-Dichlorbenzol) und dem gewünschten Nitril und geringen Mengen an Wasser. Diese organische Phase wird in der anschließenden Destillation fraktioniert.

Die Rückführung des in den nachfolgenden Destillationen wiedergewonnenen 1,2-Dichlorbenzols in die Extraktion, wie sie für die Wirtschaftlichkeit eines Verfahres erforderlich ist, hat einen nur sehr geringen Einfluß auf die Qualität der Extraktion und kann mit sehr geringen Ausschleusraten durchgeführt werden, so daß kaum Chemikalienverluste entstehen.

Führt man die Destillation der bei der Extraktion erhaltenen organischen Phase im Druckbereich zwischen etwa 1,3 bis 1015 mbar, (1 und 760 Torr), vorzugsweise zwischen etwa 13,3 bis 267 mbar, (10 and 200 Torr), aus, so gehen noch relativ geringe Mengen an Wasser über, die etwa 20 bis 80 Gewichtsprozent an Nitril enthalten, der Anteil an 1,2-Dichlorbenzol bei dieser Fraktion ist sehr gering. Die hauptsächlich aus Wasser und Nitril bestehende Fraktion wird wieder der Ausgangsmischung zugegeben, die in die Extraktionssäure eingegeben wird.

Das so erfindungsgemäß isolierte N,N-Dimethylaminoacetonitril und die daraus durch Verseifung zu gewinnende $\alpha$-Amino-carbonsäure sind wichtige Zwischenprodukte für die Herstellung von Farbstoffen, Textilhilfsmitteln, pharmazeutischen Produkten und Wirkstoffen für den Pflanzenschutz.

### Beispiel 1

2,9 kg der aus der Unsetzung von Dimethylamin, Formaldehyd und Blausäure stammenden, aus N,N-Dimethylacetonitril und Wasser bestehenden Mischung mit einem Nitril-Gehalt von etwa 48 Gewichtsprozent werden mit 10 g 1,2-Dichlorbenzol in der in Abbildung aufgezeigte Anlage umgesetzt. Dabei wird wie folgt verfahren:

In der dargestellten Anlage werden aus den Vorlagen für das Wasser/Nitril-Gemisch (1) und für 1,2-Dichlorbenzol (2) über die Pumpen (3) und (4) und die Wärmeaustauscher (11) und (12) bei 24°C 2,9 kg/h des Gemisches mit einem Gehalt von 48 Gew.—% N,N-Dimethyl-aminoacetonitril und 10 kg/h 1,2-Dichlorbenzol über Ringbrausen in den unteren bzw. oberen Teil einer pulsierten (8) Siebbodenextraktionskolonne (7) eingebracht.

Die sich in der kontinuierlichen wäßrigen Phase ausbildende disperse organische Phase durchläuft die Säule und wird an den Böden durch einen Hub des Faltenbalgs im Sumpf (5) von 20 mm bei einer Pulsationsfrequenz von 200/min ständig neu verteilt und läuft schließlich über den Überlauf (9) ab. Die wäßrige Phase durchläuft die Säule von unten nach oben (Gegenstrom) und tritt über Kopf (6) bei (10) aus. Nach Einstellung des stationären Zustands hat die Wasserphase einen Gehalt (gaschromatographisch) von 99,9 Gew.—% Wasser (Dimethylaminoacetonitril nicht mehr nachweisbar) und die organische Phase enthält 87,5 Gew.—% 1,2-Dichlorbenzol und 12,3% Nitril sowie geringe Mengen Wassers.

228,2 kg der organischen Phase werden über eine 3 m-Füllkörperkolonne bei 100 mbar (etwa 75 Torr) und einem Rücklaufverhältnis von 5:1 destilliert. Nach einem Vorlauf von 2,4 kg mit einem Wassergehalt von 17,3% 81,1% Nitril und einem geringen o-Dichlorbenzol-Anteil, der bei 50°C übergeht, wird eine Hauptfraktion von 21,4 kg bei 69°C genommen, die einen Gehalt von 99,9 Gew.—% N,N-Dimethylaminoacetonitril hat. Der Sumpf von 204,4 kg enthält außer Dichlorbenzol noch 0,1% Nitril.

### Beispiel 2

In der Anlage des Beispiels 1 werden bei 80°C und einem Pulsationshub von 10 mm bei einer Frequenz von 132/min 2,9 kg/h Wasserphase mit 48% Nitril und 10 kg/h o-Dichlorbenzol durchgesetzt. Das ablaufende Raffinat enthält 99,7% Wasser und 0,3% Nitril, der Extrakt hat Gehalte von 11,1 Gew.—% Nitril und 85,9% 1,2-Dichlorbenzol sowie 3% Wasser, das zum größten Teil in einer zweiten Phase vorliegt.

Die Destillation von 170 kg des Extraktes unter den Bedingungen wie in Beispiel 1 ergibt einen Vorlauf von 12,5 kg mit 41,5 Gew.—% Wasser, 55,1 Gew.—% Nitril und 3,4% Dichlorbenzol sowie einen Hauptlauf bei 69°C von 12,2 kg mit 99,9% Nitril. Im Sumpf von 144,9 kg ist gaschromatographisch kein Nitril mehr nachweisbar.

### Beispiel 3

In die Anlage des Beispiels 1 werden bei 23,5°C unter den gleichen Extraktionsbedingungen wie im Beispiel 2 pro Stunde 8,4 kg 1,2-Dichlorbenzol und 4,5 kg Gemisch mit 45% Nitril und 55% Wasser eingegeben. Die über Kopf ablaufende Wasserphase hat noch einen Nitril-Gehalt von 2,1 Gew.—% und die gesammelte Extraktphase enthält 23,4 Gew.—% Nitril, 76,1 Gew.—% Dichlorbenzol und 0,5% Wasser.

Bei der Destillation von 98,2 kg Extrakt wie in Beispiel 1 werden 2,1 kg Vorlauf mit 23,4 Gew.—% Wasser, 75,2 Gew.—% Nitril und einem geringen Dichlorbenzolanteil und eine Hauptfraktion von 23 kg reinen Nitrils erhalten. Der zurückbleibende Sumpf von 73 kg enthält außer Dichlorbenzol eine Spur N,N-Dimethylaminoacetonitril.

### Beispiel 4

In der Anlage des Beispiels 1 werden bei 23,5°C pro Stunde 2,9 ltr. des Nitril/Wasser-Gemisches aus Beispiel 2 mit einem Gehalt von 10 Gew.—% Natriumchlorid mit 10 kg eines Gemisches von 99,9 Gew.—% 1,2-Dichlorbenzol und 0,1 Gew.—% Dimethylaminoacetonitril unter den Bedingungen des Beispiels 2 extrahiert. Die ablaufende Wasserphase enthält 0,2 Gew.—% Nitril und der Extrakt hat Gehalte von 88 Gew.—% 1,2-Dichlorbenzol und 12 Gew.—% Nitril. Wasser ist nur in Spuren vorhanden.

Die Destillation von 205 kg des Extraktes unter den Bedingungen des Beispiels 1 ergibt nach einem Vorlauf von 1 kg mit einem Nitrilgehalt von 74,8%, 10,3 Wasser und 14,9% 1,2-Dichlorbenzol eine Hauptfraktion von 23,8 kg mit 99,9% Nitril. Der Sumpf von 180 kg hat einem Nitrilgehalt von unter 0,1%.

### Patentansprüche

1. Verfahren zur Isolierung bzw. Gewinnung von N,N-Dimethylaminoacetonitril aus den bei der Herstellung aus Dimethylamin, Blausäure und Formaldehyd anfallenden Wasser/Nitril-Gemischen durch Extraktion, dadurch gekennzeicht, daß man 1,2-Dichlorbenzol als Extraktionsmittel verwendet und die bei der Extraktion erhaltene organische Phase in einer anschließenden Destillation fraktioniert.

2. Verfahren nach Anspruch 1, dadurch gekennzeichnet, daß die Extraktion bei Temperaturen zwischen +5 und 90°C durchgeführt wird.

3. Verfahren nach Ansprüchen 1 und 2, dadurch gekennzeichnet, daß die Extraktion bei Temperaturen zwischen +15 und 30°C durchgeführt wird.

4. Verfahren nach Anspruch 1, dadurch gekennzeichnet, daß man auf 1 Gew.—Teil Wasser/Nitril-Gemisch zwischen 1,5 und 10 Gew.—Teilen 1,2-Dichlorbenzol einsetzt.

5. Verfahren nach Ansprüchen 1 und 4, dadurch gekennzeichnet, daß man auf 1 Gew. —Teil Wasser/Nitril-Gemisch zwischen 2,5 und 5 Gew.—Teilen 1,2-Dichlorbenzol einsetzt.

**Revendications**

1. Procédé pour isoler et obtenir le N,N-di-methylaminoacétonitrile par extraction de mélanges d'eau et de nitrile obtenus dans la production à partir de la diméthylamine, d'acide cyanhydrique et de formaldèhyde, caractérisé en ce qu'on utilise le 1,2-dichlorobenzène commme agent d'extraction et en ce que la phase organique obtenue lors de l'extraction est fractionnée au cours d'une distillation subséquente.

2. Procédé suivant la revendication 1, caractérisé en ce que l'extraction est conduite à des températures comprises entre +5 et 90°C.

3. Procédé suivant les revendications 1 et 2, caractérisé en ce que l'extraction est conduite à des températures comprises entre +15 et 30°C.

4. Procédé suivant la revendication 1, caractérisé en ce qu'on utilise 1,5 à 10 parties en poids de 1,2-dichlorobenzène par partie en poids de mélange d'eau et de nitrile.

5. Procédé suivant les revendications 1 et 4, caractérisé en ce qu'on utilise 2,5 à 5 parties en poids de 1,2-dichlorobenzène par partie en poids de mélange d'eau et de nitrile.

**Claims**

1. A process for the isolation or recovery of N,N-dimethylaminoacetonitrile from water/nitrile mixtures, obtained during the preparation from dimethylamine, hydrocyanic acid and formaldehyde, by means of extraction characterised in that 1,2-dichlorobenzene is used as the extraction agent and the organic phase obtained during the extraction is fractionated by subsequent distillation.

2. A process according to claim 1, characterised in that the extraction is conducted at temperatures between +5 and 90°C.

3.a A process according to claims 1 and 2, characterised in that the extraction is carried out at temperatures between +15 and 30°C.

4. A process according to claim 1, characterised in that between 1.5 and 10 parts by weight of 1,2-dichlorobenzene are used per part by weight of the water/nitrile mixture.

5. A process according to claims 1 and 4, characterised in that between 2.5 and 5 parts by weight of 1,2-dichlorobenzene are used per part by weight of the water/nitrile mixture.